# EUROPÄISCHE PATENTSCHRIFT

(11) **EP 1 069 884 B1**
(45) Veröffentlichungstag und Bekanntmachung des Hinweises auf die Patenterteilung: **25.09.2002**
(21) Anmeldenummer: 99913311.9
(22) Anmeldetag: 27.03.1999
(51) Int. Cl.: A61K 7/48

(54) **RIBONUCLEIN- ODER DESOXYRIBONUCLEINSAÜREN ENTHÄLTENDES KOSMETISCHE ODER PHARMAZEUTISCHE MITTEL**
COSMETIC OR PHARMACEUTICAL AGENTS CONTAINING RIBONUCLEIC OR DESOXYRIBONUCLEIC ACIDS
PRODUITS COSMETIQUES OU PHARMACEUTIQUES RENFERMANT DES ACIDES RIBONUCLEIQUES OU DESOXYRIBONUCLEIQUES

(30) Priorität: 06.04.1998 DE 19815090
(43) Veröffentlichungstag der Anmeldung: 24.01.2001
(73) Patentinhaber: Cognis Deutschland GmbH & Co. KG, 40589 Düsseldorf (DE)
(72) Erfinder: HÖRNER, Viola, D-40593 Düsseldorf (DE); KÜHNE, Sabine, D-42781 Haan (DE); WACHTER, Rolf, D-40595 Düsseldorf (DE)
(86) Internationale Anmeldenummer: EP9902115
(87) Internationale Veröffentlichungsnummer: WO99051200

(56) Entgegenhaltungen:
- EP-A- 0 283 893
- EP-A- 0 379 846
- EP-A- 0 416 677
- CH-A- 678 489
- CH-A- 686 997
- FR-A- 2 511 243
- FR-A- 2 543 438
- DATABASE WPI Week 9239 Derwent Publications Ltd., London, GB; AN 92-320239 XP002110957 "Liquid composition used in toiletries material-comprises hydrophilic surfactant, sterol, alpha glycerylether, macromolecular electrolyte and e.g. guar gum" & JP 04 224886 A (KOSE KK) 14. August 1992 (1992-08-14)

## Beschreibung

### Gebiet der Erfindung

Die Erfindung betrifft kosmetische Mittel enthaltend (Desoxy)ribonucleinsäuren, Emulgatoren, Ölkörper und Naturstoffe sowie die Verwendung von (Desoxy)Ribonucleinsäuren zur Herstellung kosmetischer undloder pharmazeutischer Mittel.

### Stand der Technik

Kosmetische und/oder pharmazeutische Zubereitungen stehen dem Verbraucher haute in einer Vielzahl von Kombinationen zu Verfügung. Dennoch besteht im Markt weiterhin das Bedürfnis nach Produkten mit einem verbesserten Leistungsspektrum. Hierbei sind insbesondere Hautverträglichkeit sowie der Einsatz natürlicher Produkte beim Kunden gefragt Daneben wäre es wünschenswert, durch Kombination mit bereits bekannten Wirkstoffen, deutlich bessere Produkte zu erhalten.

Der Einsatz von Nucleinsäuren als Wirkstoffe in der Kosmetik ist bekannt. So werden beispielsweise in der französischen Patentanmeldung **FR-A1 2511253** Haut- und Sonnenschutzmittel mit einem Gehalt an hochpolymerisierter DNA vorgeschlagen. Aus der japanischen Offenlegungsschrift **JP-A2 62/096404** (Kanebo) sind kosmetische Zusammensetzungen mit Nucleinsäuren und Diisopropylamindichloracetat bekannt. Gegenstand der französischen Patentschrift **FR-B1 2620024** (Soc. d'Etudes Dermatologiques) sind Zubereitungen, enthaltend Nucleinsäurederivate als Radikalfänger. Beispiele sind Adenin, Guanosin, Xanthin, Hypoxanthin, Uracil und Ribonucleinsäure. Die Patentanmeldung EP-A-0283893 offenbart Zubereitungen enthaltend Ölkörper, Hyaluronsäure und Ribonucleinsäure. In der internationalen Patentanmeldung **WO 95/01773** (Boston University) wird ein Verfahren zur Stimulation der Pigmentproduktion beschrieben, bei dem man DNA-Fragmente, bevorzugt Dinucleotide, in liposomaler Form in die Epidermis transportiert. Gegenstand der deutschen Patentanmeldung **DE-A1 4323615** sind schließlich Zusammensetzungen mit einem Gehalt an Nucleinsäuren und deren Fragmenten als Anti-Ageing- und Sonnenschutzcremes. Die **JP A 6-271451** beschreibt Zubereitungen für die Behandlung der Haut, die 4-tert.-Butyl-4'-Methoxybenzoytmethan in Kombination mit einer Nukleinsäure enthalten, die **JP A 6-100426** beschreibt die Kombination von Hyaluronidaseinhibitor und Desoxyribonucleinsäuren. In **AT E 28 399 B** wird ein Verfahren zur Herstellung einer Zusammensetzung aus zerkleinertem Getreide und Sojakeimen beschrieben, das Desoxyribonucleinsäuren aus Getreide und/oder Sojakeimen enthält.

Die komplexe Aufgabe der Erfindung hat darin bestanden, kosmetische und/oder pharmazeutische Zubereitungen zur Verfügung zu stellen, die den hohen Anforderungen an Phasenstabilität, Lagerbeständigkeit, Verträglichkeit gegenüber empfindlicher Haut gerecht werden und möglichst zusätzlich noch deutlich verbesserte hautpflegende und schützende Eigenschaften aufweisen.

### Beschrelbung der Erfindung

Gegenstand der Erfindung sind kosmetische und/oder pharmazeutische Zubereitungen, enthaltend
(a) 0,5 bis 2,5 Gew.% (Desoxy)Ribonucleinsäuren,
(b) 0,1 bis 10 Gew.% Emulgatoren und
(c) 1 bis 90 Gew.% Ölkörper und
(d) 0,001 bis 5 Gew.% Panthenol.

Überraschenderweise wurde gefunden, daß durch den Einsatz von (Desoxy)Ribonucleinsäuren Produkte erhalten werden, die gute pflegende und schützende Eigenschaften sowie gleichzeitig hohe Hautverträglichkeit aufweisen. Überraschenderweise wurde gefunden, daß durch die Verwendung von (Desoxy)Ribonucleinsäuren besonders stabile Emulsionen erhalten werden. Des weiteren wurde gefunden, daß der Einsatz von (Desoxy)Ribonucleinsäuren Eigenschaften von Panthenol in unerwarteter Weise verstärkt, hierzu zählen beispielsweise die pflegenden, feuchtigkeitsbindenden und entzündungshemmenden Eigenschaften. Die antioxidative Kapazität der (Desoxy)Ribonucleinsäuren schützt zum einen die Haut vor entzündlichen Reaktionen, zum anderen werden gleichzeitig die kosmetischen Mittel vor oxidativem Abbau (Verderb) geschützt.

### (Desoxy)Ribonucleinsäuren

Unter (Desoxy)Ribonucleinsäuren (DNA bzw. RNA) werden hochmolekulare, fadenförmige Polynucleotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribofuranose bzw. D-Ribofuranose aufgebaut werden. Als Nudeobasen können die DNA bzw. RNA die Purinderivate Adenin und Guanin sowie die Pyrimidine Cytosin und Thymin bzw. Uracil enthalten. In den Nudeinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzel-strang-DNA bzw. -RNA. In der Regel liegt die DNA doppelsträngig vor (Ausbildung von Wasserstoffbrückenbindungen zwischen den entsprechenden Basen) und die RNA einzelsträngig. Je nach Behandlung der Nucleinsäuren, können sowohl DNA als auch RNA in Form von Doppelsträngen undloder Einzelsträngen vorliegen. Auch Heterodimere (Doppelstrang zwischen DNA und RNA) sind möglich. Unter dem Begriff (Desoxy)Rbonucleinsäuren im Sinne der vorliegenden Erfindung werden sowohl doppel- als auch einzelsträngige (Desoxy)Ribonucleinsäuren verstanden, desweiteren Mischungen aus Einzelsträngen und Doppelsträngen. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton erreichen. Im Sinne der Erfindung werden konzentrierte DNA bzw. RNA-Lösungen eingesetzt, die sich durch ein flüssigkristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 1.000 bis 5.000.000 Dalton aufweisen. Besonders bevorzugt ist der Einsatz von einzelsträngigen Desoxyribonucleinsäuren marinen Ursprungs mit einem Molekulargewicht im Bereich von 1.000 bis 1.000.000, insbesondere von 10.000 bis 100.000 Dalton. Die (Desoxy)Ribonucleinsäuren können in den erfindungsgemäßen Mitteln in Mengen von 0,001 bis 2,5, vorzugsweise 0,1 bis 0,5 Gew.-% - bezogen auf das Mittel - enthalten sein.

### Pflanzliche Wirkstoffe

Eins Vielzahl von pflanzlichen Wirkstoffen hat beruhigende, pflegende, feuchtigkeitsspendende, zum Teil auch entzündungshemmende, UV-absorbierende, hautaufhellende und/oder selbstbräunende Wirkung auf die Haut. Aus diesem Grund ist der Einsatz dieser Stoffe in kosmetischen und/oder pharmazeutischen Zubereitungen von großer Bedeutung. Ein Beispiel für einen pflanzlichen Stoff mit diesem Leistungsspektrum ist Panthenol. Die Gesamtmenge an Naturstoffen im Endprodukt liegt in der Regel zwischen 0,001 bis 25 Gew.% - bezogen auf das Mittel -, vzw. 0,001 bis 5 Gew.%. Besonders bevorzugt ist ein Einsatz von 0,01 bis 2,5 Gew.%.

### Emulgatoren

Als **Emulgatoren** (Komponente b) kommen beispielsweise nichtionogene Tenside aus mindestens einer der folgenden Gruppen in Frage:
(1b) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2b) C_{12/18}-Fettsäuremono- und -diester von Anlagerungsprodukten von 1 bis 30 Mol Ethylenoxid an Glycerin;
(3b) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4b) Alkylmono- und -oligoglycoside mit 8 bis 22 Kohlenstoffatomen im Alkylrest und deren ethoxylierte Analoga;
(5b) Anlagerungsprodukte von 15 bis 60 Mol Ethylenoxid an Ricinusöl und/oder gehärtetes Ricinusöl;
(6b) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7b) Anlagerungsprodukte von 2 bis 15 Mol Ethylenoxid an Ricinusöl und/oder gehärtetes Ricinusöl;
(8b) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9b) Mono-, Di- und Trialkylphosphate sowie Mono-, Di- und/oder Tri-PEG-alkylphosphate und deren Salze;
(10b) Wollwachsalkohole;
(11b) Polysiloxan-Polyalkyl-Polyether-Copolymere bzw. entsprechende Derivate;
(12b) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13b) Polyalkylenglycole
(14b) Betaine
(15b) Esterquats.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind. Desweiteren können als anionische Emulgatoren Alkylethersulfate, Monoglyceridsulfate, Fettsäuresulfate, Sulfosuccinate und/oder Ethercarbonsäuren eingesetzt werden. Die Emulgatoren können in den erfindungsgemäßen Mitteln in Mengen von 0,1 bis 10, vorzugsweise 1 bis 5 Gew.-% - bezogen auf das Mittel - enthalten sein.

### Ölkörper

Als Ölkörper (Komponente c) kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestem mit Polyolen, Siliconöle und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Als Ölkörper können weiterhin Kohlenwasserstoffe wie Squalan und Squalen eingesetzt werden. Die Ölkörper können in den erfindungsgemäßen Mitteln in Mengen von 1 bis 90, vorzugsweise 5 bis 80 und insbesondere 10 bis 50 Gew.-% - bezogen auf das Mittel - enthalten sein.

### Vitamine

In einer bevorzugten Ausführungsform enthalten die erfindungsgemäßen Zubereitungen weiterhin Vitamine. Hierbei sind besonders bevorzugt Vitamine, die ausgewählt sind aus der Gruppe, die gebildet wird von Tocopherolen, Ascorbinsäure, Carotinoiden, Biotin und Vitamin A.

Unter Tocopherolen versteht man in 2-Stellung mit einem 4,8,12,-Trimethyltridecyl-Rest substituierte Chroman-6-ole (3,4-Dihydro-2*H*-benzopyran-6-ole). Unter dem Begriff "Tocopherole" werden in Sinne dieser Anmeldung auch die Biochinone, d.h. zu den polyprenylierten 1,4-Benzo- bzw. Naphthochinonen gehörenenden Plastochinone, Tocopherolchinone, Ubichinone, Bovichinone, K-Vitamine und Menachinone (2-Methyl-1,4-naphthochinone) zusammengefaßt. In Frage kommen insbesondere α-, β-, γ- und δ-Tocopherole die der allgemeinen Formel **(I)** folgen (R = Wasserstoff oder Methyl), die ε-Tocopherole der allgemeinen Formel **(II)**, die noch über die ursprüngliche ungesättigte Prenylseitenkette verfügen, sowie die α-Tocopherolchinone und -hydrochinone der allgemeinen Formel **(III)**, bei denen das Pyran-Ringsystem geöffnet ist.

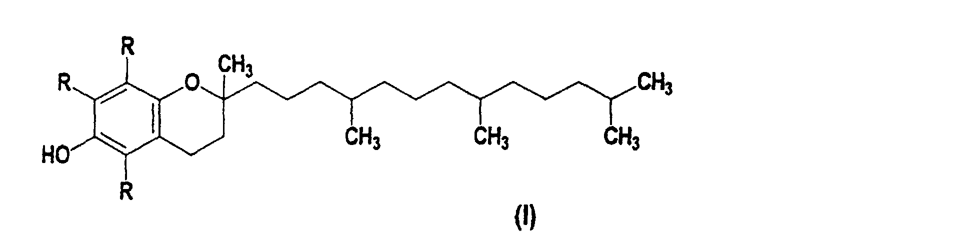

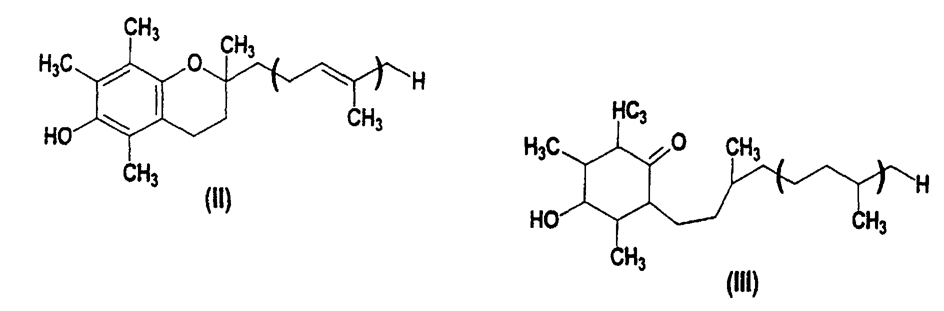

Neben den Tocopherolen kommen auch deren Derivate, insbesondere Ester mit Carbonsäuren, wie beispielsweise Tocopherolacetat oder -palmitat in Frage. Die Tocopherole können in den erfindungsgemäßen Mitteln in Mengen von 0,001 bis 20,0 Gew.-%, vorzugsweise von 0,2 bis 2,5 Gew.-% - bezogen auf das Mittel - enthalten sein.

### Ascorbinsäure

Ascorbinsäure (Vitamin C) wird als wasserlösliches Vitamin in einer Vielzahl von kosmetischen Produkten eingesetzt. Hier dient es in der Regel als natürliches Antioxidans, oft in Kombination mit fettlöslichen Vitaminen wie Tocopherolen. Ascorbinsäure wird in der Regel als freie Säure oder in Form von Ascorbylpalmitat oder Ascorbylacetat eingesetzt. In der Regel ist Ascorbinsäure in den erfindungsgemäßen Mitteln in Mengen von 0,05 bis 1,0 Gew.-%, vorzugsweise von 0,1 bis 0,5 Gew.-% - bezogen auf das Mittel - enthalten.

### Carotinoide

Die Carotinoide an sich haben keinen Vitamincharakter, sie werden aufgrund der Funktion der β-Carotins als Provitamin A jedoch im Sinne dieser Anmeldung unter den Begriff der Vitamine miterfaßt. Unter Carotinoiden werden von Carotin abgeleitete, kohlenstoffhaltige Substanzen zusammengefaßt, deren Grundgerüst aus acht Isopren-Einheiten (Tetraterpen) besteht. Die Sauerstoffhaltigen Derivate der Carotinoide werden als Xanthophylle bezeichnet. Die 3 bedeutendsten Isomere des Carotins sind: α-, β- und γ- Carotin. Alle 3 besitzen das gleiche Grundgerüst mit 9 konjugierten Doppelbindungen, 8 Methyl-Verzweigungen und einer β-lonon Struktur am Molekülende, β-Carotin ist das in der Natur am häufigsten vorkommende Carotinoid. α-Carotin leitet sich formal vom β-Carotin durch Verschiebung der Doppelbindung in die 4',5'-Stellung ab, im γ-Carotin ist der rechte Ring zwischen C-1'und C-6'geöffnet, wodurch eine neue Doppelbindung entsteht. Weitere Carotinoide sind δ-, ξ-, und ε- Carotin. Das wichtigste Carotin Isomer ist β-Carotin, das eigentliche Provitamin A, das im tierischen Organismus in zwei Moleküle Retinal gespalten wird. Carotinoide können in den erfindungsgemäßen Mitteln in Mengen von 0,05 bis 1,0 Gew.-%, vorzugsweise von 0,1 bis 0,5 Gew.-% - bezogen auf das Mittel - enthalten sein.

### Biotin

Biotin (D-cis-Hexahydro-2-oxothienol(3,4-*d*]imidazol-4-valeriansäure), gehört zu den Vitaminen der B Gruppe (veraltete Bezeichnung für Biotin ist Vitamin H). Biotin kann in den erfindungsgemäßen Mitteln in Mengen von 0,001 bis 2,5 Gew.-%, insbesondere in Mengen von 0,01 bis 1,0 Gew.-%, -bezogen auf das Mittel-, enthalten sein.

### Vitamin A

Unter Vitamin A im Sinne der vorliegenden Erfindung werden Retinol, Retinal (synonym Retinaldehyd) und Retinsäure sowie deren Stereoisomere und Ester zusammengefaßt, aber auch alle Verbindungen, die sich von der Stammverbindung Retinol (all-trans Retinol) ableiten, auch synthetisch hergestellte, die nicht in der Natur vorkommen (sog. Retinoide). Vitamin A kann in den erfindungsgemäßen Mitteln in Mengen von 0,001 bis 0,01 Gew.-% -bezogen auf das Mittel- enthalten sein.

Die Gesamtmenge an Vitaminen im Endprodukt liegt in der Regel zwischen 0,001 bis 25 Gew.-% - bezogen auf das Mittel-, vorzugsweise 0,01 bis 5 Gew.-%. Besonders bevorzugt ist ein Einsatz Von 0,1 bis 2,5 Gew.-%.

### Gewerbliche Anwendbarkeit

Die erfindungsgemäßen Mittel zeichnen sich durch gute Schutz- und Pflegewirkung der Haut bei besonders vorteilhafter hautkosmetischer Verträglichkeit aus. Insbesondere die Eigenschaften der Naturstoffe wird in den erfindungsgemäßen Zubereitungen auf unerwartete Weise erhöht. Dabei können die erfindungsgemäßen Mittel beispielsweise als Creme, Milch, Öl, Gele oder auch Lippenstift vorliegen. Typische Zubereitungen enthalten 0.5 % bis 2,5, % - bezogen auf das Mittel- der Komponente (a) (Desoxy)Ribonucleinsäuren.

Die Zubereitungen haben folgende Zusammensetzung
(a) 0,5 bis 2,5 Gew.% (Desoxy)Ribonucleinsäuren,
(b) 0,1 bis 10 Gew.% Emulgatoren und
(c) 1 bis 90 Gew.% Ölkörper und
(d) 0,001 bis 5 Gew.% Panthenol.

Die Angabe der Gew.-% bezieht sich dabei auf das fertige Mittel. Innerhalb der genannten Konzentrationsbereiche werden besonders stabile und feinteilige Emulsionen erhalten.

Als weitere Inhaltsstoffe können sie in untergeordneten Mengen weitere, mit den anderen Inhaltsstoffen kompatible Tenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofem die nicht-ionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

Die erfindungsgemäßen Mittel können femer als weitere Hilfs- und Zusatzstoffe Co-Emulgatoren, Überfettungsmittel, Stabilisatoren, Wachse, Konsistenzgeber, Verdickungsmittel, Kationpolymere, biogene Wirkstoffe, Konservierungsmittel, UV-Lichtschutzfilter, Hydrotrope, Solubilisatoren, Farb- und Duftstoffe enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.
Als **Periglanzwachse** kommen beispielsweise in Frage: Alkylenglycolester, speziell Ethylenglycoldistearat: Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohienstoffatomen und 2 bis 10 Hydroxylgruppen sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/lsobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere. Vinylpyrrolidon/Dimethylaminoethylmethacrylat/Vinylcaprolactam-Terpolymere sowie gegebenenfalls derivatisierte Celluloseether und Silicone in Frage.

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Phytantriol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können femer **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutem und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, ∝-Hexylzimtaldehyd, Geraniol, Benzylaceton, Cyclamenaldehyd, Linaloöl, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

Ein weiterer Gegenstand der Erfindung betrifft die Verwendung von Zubereitungen, enthaltend
(a) 0,5 bis 2,5 Gew.% (Desoxy)Ribonucleinsäuren,
(b) 0,1 bis 10 Gew.% Emulgatoren und
(c) 1 bis 90 Gew.% Ölkörper und
(d) 0,001 bis 5 Gew.% Panthenol
zur Herstellung von kosmetischen und/oder pharmazeutischen Zubereitungen. Diese Zubereitungen können in üblichen Formulierungen, wie Cremes, Lotionen, Milch, Ölen, aber auch Gelen und Lippenstiften vorliegen.

### Rezepturbeispiele

**Tab. 1**

| **Softcreme Rezeptur K1** (Alle Angaben in Gew.-% bez. auf das kosmetische Mittel) | |
|---|---|
| | K1 |
| Glyceryl Stearate (and) Ceteareth-12/20 (and) Cetearyl Alcohol (and) Cetyl Palmitate | 5,0 |
| Decyl Oleate | 3,0 |
| Cetearyl Isononanoate | 3,0 |
| Glycerin (86 Gew.-%ig) | 3,0 |
| Desoxyribonucleinsäure ¹⁾ | 0,5 |
| Panthenol | 0,5 |
| Wasser | Ad 100 |

**Tab. 2**

| **W/O Bodylotion Rezeptur K2** (Alle Angaben in Gew.-% bez. auf das kosmetische Mittel) | |
|---|---|
| | K2 |
| Ricinusöl hydriert + 7 EO | 7,0 |
| Decyl Oleate | 7,0 |
| Cetearyl Isononanoate | 7,0 |
| Glycerin (86 Gew.-%ig) | 5,0 |
| Desoxyribonucleinsäure ¹⁾ | 1,5 |
| MgSO₄ ∗ 7 H₂O | 0,5 |
| Panthenol | 0,5 |
| Wasser | Ad 100 |

**Tab. 3**

| **Lippenstift (pflegend) Rezeptur L 1** (Alle Angaben in Gew.-% bez. auf das kosmetische Mittel) | |
|---|---|
| INCl Bezeichnung | L1 |
| Fettsäuretriglycerid (C8-C10) | 14,0 |
| Propylene glycol dicaprylate/ caprate | 6,0 |
| Octyldodecanol | 17,0 |
| Candelilla wax | 5,0 |
| Camauba wax | 7,0 |
| Cera alba bees wax | 5,0 |
| Glyceryl laurate | 3,0 |
| Polyglyceryl-2-Dipolyhydroxystearate | 4,0 |
| Castor oil (Ricinusfettsäuretriglycerid) | 18,0 |
| Desoxyribonucleinsäure ¹⁾ | 0,5 |
| Panthenol | 0,5 |
| Wasser | ad 100 |

| | |
|---|---|
| ¹⁾ Desoxyribonucleinsäure: Molekulargewicht ca. 70000, Reinheit (bestimmt durch spektrophotometrische Messung der Absorption bei 260 nm sowie 280 nm): mindestens 1,7. | |

## Patentansprüche

1. Kosmetische und/oder pharmazeutische Zubereitungen, enthaltend
| | | |
|---|---|---|
| (a) | 0,5 bis 2,5 Gew. % | (Desoxy)ribonucleinsäuren |
| (b) | 0,1 bis 10 Gew. % | Emulgatoren |
| (c) | 1 bis 90 Gew. % | Ölkörper und |
| (d) | 0,001 bis 5 Gew. % | Panthenol |

2. Kosmetische und/oder pharmazeutische Zubereitungen nach Anspruch 1, **dadurch gekennzeichnet, dass** sie als Komponente (a) (Desoxy)ribonucleinsäuren eines Molekulargewichtes von 1 000 bis 5 000 000 enthalten.

3. Kosmetische und/oder pharmazeutische Zubereitungen nach den Ansprüchen 1 und 2, **dadurch gekennzeichnet, dass** sie als Komponente (a) (Desoxy)ribonucleinsäuren eines Molekulargewichtes von 10 000 bis 100 000 enthalten.

4. Kosmetische und/oder pharmazeutische Zubereitungen nach den Ansprüchen 1 bis 3, **dadurch gekennzeichnet, dass** sie Ölkörper enthalten, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estem von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen C₆-C₂₂-Fettalkoholcarbonaten, Guerbetcarbonaten, Estem der Benzoesäure mit linearen und/oder verzweigten C₆-C₂₂-Alkoholen, Dialkylethern, Ringöffnungsprodukten von epoxidierten Fettsäureestern mit Polyolen, aliphatischen bzw. naphthenischen Kohlenwasserstoffen sowie Siliconölen.

5. Kosmetische und/oder pharmazeutische Zubereitungen nach den Ansprüchen 1 bis 4, **dadurch gekennzeichnet, dass** sie Emulgatoren enthalten, die ausgewählt sind aus der Gruppe, die gebildet wird von
(b1) Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe
(b2) C_{12/18}-Fettsäuremono- und -diestern von Anlagerungsprodukten von 1 bis 30 Mol Ethylenoxid an Glycerin
(b3) Glycerinmono- und -diestern und Sorbitanmono- und -diestem von gesättigten und ungesättigten Fettsäuren mit 6 bis 22 Kohlenstoffatomen und deren Ethylenoxidanlagerungs-Produkten
(b4) Alkylmono- und -oligoglycosiden mit 8 bis 22 Kohlenstoffatomen im Alkylrest und deren ethoxylierten Analoga
(b5) Anlagerungsprodukten von 15 bis 60 Mol Ethylenoxid an Ricinusöl und/oder gehärtetes Ricinusöl
(b6) Polyol- und insbesondere Polyglycerinestern
(b7) Anlagerungsprodukten von 2 bis 15 Mol Ethylenoxid an Ricinusöl und/oder gehärtetes Ricinusöl
(b8) Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden
(b9) Mono- Di- und Trialkylphosphaten sowie Mono-, Di- und/oder Tri-PEG-alkylphosphaten
(b10) Wollwachsalkoholen
(b11) Polysiloxan-Polyalkyl-Polyether-Copolymeren bzw. entsprechende Derivaten
(b12) Mischestem aus Pentaerythrit, Fettsäuren, Citronensäure und Fettalkohol und/oder Mischester von Fettsäuren mit 6 bis 22 Kohlenstoffatomen, Methylglucose und Polyolen
(b13) Polyalkylenglycolen
(b14) Betainen
(b15) Esterquats

6. Verwendung von kosmetischen und/oder pharmazeutischen Zubereitungen, enthaltend
| | | |
|---|---|---|
| (a) | 0,5 bis 2,5 Gew. % | (Desoxy)ribonucleinsäuren |
| (b) | 0,1 bis 10 Gew. % | Emulgatoren, |
| (c) | 1 bis 90 Gew. % | Ölkörper und |
| (d) | 0,001 bis 5 Gew. % | Panthenol. |
zur Herstellung von kosmetischen und/oder pharmazeutischen Zubereitungen.

## Claims

1. Cosmetic and/or pharmaceutical preparations containing
| | |
|---|---|
| (a) | 0.5 to 2.5% by weight (deoxy)ribonucleic acids |
| (b) | 0.1 to 10% by weight emulsifiers, |
| (c) | 1 to 90% by weight oil components and |
| (d) | 0.001 to 5% by weight panthenol. |

2. Cosmetic and/or pharmaceutical preparations as claimed in claim 1, **characterized in that** they contain (deoxy)ribonucleic acids with a molecular weight of 1,000 to 5,000,000 as component (a).

3. Cosmetic and/or pharmaceutical preparations as claimed in claims 1 and 2, **characterized in that** they contain (deoxy)ribonucleic acids with a molecular weight of 10,000 to 100,000 as component (a).

4. Cosmetic and/or pharmaceutical preparations as claimed in claims 1 to 3, **characterized in that** they contain oil components selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C_{6ν13} carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/tri- glyceride mixtures based on C₆₋₁₈ fatty acids, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring opening products of epoxidized fatty acid esters with polyols, aliphatic or naphthenic hydrocarbons and silicone oils.

5. Cosmetic and/or pharmaceutical preparations as claimed in claims 1 to 4, **characterized in that** they contain emulsifiers selected from the group consisting of
(b1) products of the addition of 2 to 30 moles of ethylene oxide and/or 0 to 5 moles of propylene oxide onto linear fatty alcohols containing 8 to 22 carbon atoms, onto fatty acids containing 12 to 22 carbon atoms and onto alkylphenols containing 8 to 15 carbon atoms in the alkyl group;
(b2) C_{12/18} fatty acid monoesters and diesters of products of the addition of 1 to 30 moles of ethylene oxide onto glycerol;
(b3) glycerol monoesters and diesters and sorbitan monoesters and diesters of saturated and unsaturated fatty acids containing 6 to 22 carbon atoms and ethylene oxide adducts thereof;
(b4) alkyl mono- and oligoglycosides containing 8 to 22 carbon atoms in the alkyl group and ethoxylated analogs thereof;
(b5) adducts of 15 to 60 moles of ethylene oxide with castor oil and/or hydrogenated castor oil;
(b6) polyol esters and, in particular, polyglycerol esters;
(b7) products of the addition of 2 to 15 moles of ethylene oxide onto castor oil and/or hydrogenated castor oil;
(b8) partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols, alkyl glucosides and polyglucosides;
(b9) mono-, di- and trialkyl phosphates and mono-, di- and/or tri-PEG-alkyl phosphates and salts thereof;
(b10) wool wax alcohols;
(b11) polysiloxane/polyalkyl polyether copolymers and corresponding derivatives;
(b12) mixed esters of pentaerythritol, fatty acids, citric acid and fatty alcohol and/or mixed esters of fatty acids containing 6 to 22 carbon atoms, methyl glucose and polyols;
(b13) polyalkylene glycols;
(b14) betaines
(b15) esterquats.

6. The use of cosmetic and/or pharmaceutical preparations containing
| | |
|---|---|
| (a) | 0.5 to 2.5% by weight (deoxy)ribonucleic acids, |
| (b) | 0.1 to 10% by weight emulsifiers, |
| (c) | 1 to 90% by weight oil components and |
| (d) | 0.001 to 5% by panthenol |
for the production of cosmetic and/or pharmaceutical preparations.

## Revendications

1. Préparations cosmétiques et/ou pharmaceutiques qui contiennent :
(a) de 0,5 à 2,5 % en poids d'acides (désoxy)ribonucléiques,
(b) de 0,1 à 10 % en poids d'agents émulsionnants,
(c) de 1 à 90 % en poids de composés huileux et
(d) de 0.001 à 5 % en poids de panthénol.

2. Préparations cosmétiques et/ou pharmaceutiques selon la revendication 1,
**caractérisées en ce qu'**
elles contiennent composant (a) des acides (désoxy)ribonucléiques d'un poids moléculaire allant de 1000 à 5 000 000.

3. Préparations cosmétiques et/ou pharmaceutiques selon les revendications 1 et 2,
**caractérisées en ce que**
comme composant (a) elles contiennent des acides (désoxy)ribonucléiques d'un poids moléculaire allant de 10 000 à 100 000.

4. Préparations cosmétiques et/ou pharmaceutiques selon l'une quelconque des revendications 1 à 3,
**caractérisées en ce qu'**
elles contiennent des composée huileux qui sont choisis dans le groupe formé des alcools de Guerbet à base d'alcools gras ayant de 6 à 18 atomes de carbone, d'esters d'acide gras linéaires en C₆ à C₂₂ avec des alcools gras linéaires en C₆-C₂₂. des esters d'acides carboxyliques ramifiés en C₆-C₁₃ avec des alcools gras linéaires en C₆-C₂₂, des esters d'acide gras linéaire en C₆-C₂₂, avec des alcools ramifiés, des esters d'acide gras linéaire et/ou ramifié avec des alcools plurifonctionnels et/ou des alcools de Guerbet, des triglycérides à base d'acides gras en C₆-C₁₀, des mélanges liquides de mono-,/di-/ et triglycérides à base d'acide gras en C₆-C₁₈, des esters d'alcool gras en C₆-C₂₂ et/ou d'alcools de Guerbet avec des acides carboxyliques aromatiques, des esters d'acides dicarboxyliques en C₂-C₁₂ avec des alcools linéaires ou ramifiés ayant de 1 à 12 atomes de carbone, ou avec des polyols ayant de 2 à 10 atomes de carbone et de 2 à 6 groupes hydroxyle, des huiles végétales, des alcools primaires ramifiés, des cyclohexanes substituée, des carbonates d'alcools gras en C₆-C₂₂ linéaires, des carbonates de Guerbet, des esters de l'acide benzoïque avec des alcools linéaires et/ou ramifiés en Ce à C₂₂, des éthers dialkiliques, des produits d'ouverture de cycle d'esters d'acide gras époxydés avec des polyols, des hydrocarbures aliphatiques ou naphténiques ainsi que des huiles de silicone.

5. Préparations cosmétiques et/ou pharmaceutiques selon l'une quelconque des revendications 1 à 4,
**caractérisées en ce qu'**
elles contiennent comme agents émulsionnants, ceux choisis dans le groupe formé par :
(b1) les produits d'addition de 2 à 30 mol d'oxyde d'éthylène et/ou de 0 à 5 mol d'oxyde de propylène sur des alcools gras linéaires ayant de 8 à 22 atomes de C, sur des acides gras ayant de 12 à 22 atomes de carbone et sur des alkylphénols ayant de 8 à 15 atomes de carbone dans le groupe alkyle,
(b2) des mono- et des diesters d'acides gras en C₁₂-C₁₈ de produits d'addition de 1 à 30 mol d'oxyde d'éthylène sur le glycérol,
(b3) des mono- et des diesters de glycérol et des mono- et de diesters de sorbitane d'acides gras saturés et non saturés ayant de 6 à 22 atomes de carbone et leur produit d'addition d'oxyde d'éthylène,
(b4) des alkylmono- et oligoglycosides ayant de 8 à 22 atomes de carbone dans le reste alkyle et leurs analogues éthoxyles,
(b5) des produits d'addition de 15 à 60 mol d'oxyde d'éthylène sur l'huile de ricin et/ou l'huile de ricin durcie,
(b6) des esters de polyol et en particulier des esters de polyglycerol,
(b7) produits d'addition de 2 à 15 mol l'oxyde d'éthylène sur de l'huile de ricin ou de l'huile de ricin durcie,
(b8) des esters partiels à base d'acides gras en C₆/C₂₂ non saturés ou saturés, ramifiés, linéaires, de l'acide ricinoléïque ainsi que de l'acide 12-hydroxy stéarique et du glycérol, du polyglycérol, du pentaérythritol, du dipentaérythritol, des alcools de sucre, des alkylglucosides ainsi que des polyglucosides,
(b9) des mono-, di- et trialkylphosphates ainsi que des mono-, di- et/ou Trt-PEG-alkyl phosphates,
(b10) des alcools de cire de laine,
(b11) des copolymères polysiloxane-polyalkyl-polyéther ou des dérivés correspondants,
(b12) des esters mixtes à base de pentaérythritol, d'acides gras, d'acide citrique, et d'alcool gras et ou des esters mixtes d'acide gras ayant de 6 à 22 atomes de carbone, de méthylglucose et de polyols,
(b13) des polyalkylèneglycols,
(b14) des betaïnes,
(b15) des esterquats.

6. Utilisation de préparations cosmétiques et/ou pharmaceutiques contenant :
(a) de 0,5 à 2,5 % en poids d'acides (désoxy)ribonucléiques,
(b) de 0,1 à 10 % en poids d'agents émulsionnants,
(c) de 1 à 90 % en poids de composés huileux et
(d) de 0,001 à 5 % en poids de panthenol en vue de la fabrication de préparations cosmétiques et/ou pharmaceutiques.
